(19) **Europäisches Patentamt**
**European Patent Office**
**Office européen des brevets**

(11) **EP 4 349 969 A1**

(12) **EUROPEAN PATENT APPLICATION**
published in accordance with Art. 153(4) EPC

(43) Date of publication:
**10.04.2024 Bulletin 2024/15**

(21) Application number: **22816452.1**

(22) Date of filing: **31.05.2022**

(51) International Patent Classification (IPC):
*C12N 5/0783* (2010.01)    *C07K 14/47* (2006.01)
*C07K 14/705* (2006.01)    *C07K 14/54* (2006.01)
*C07K 14/715* (2006.01)    *C12N 5/09* (2010.01)

(52) Cooperative Patent Classification (CPC):
**C07K 14/47; C07K 14/54; C07K 14/705;**
**C07K 14/715; C12N 5/06**

(86) International application number:
**PCT/KR2022/007782**

(87) International publication number:
**WO 2022/255793 (08.12.2022 Gazette 2022/49)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **01.06.2021 KR 20210070778**

(71) Applicants:
• **Vaxcell-Bio**
**Jeollanam-do 58141 (KR)**
• **Kongju National University Industry-University**
**Cooperation Foundation**
**Gongju-si, Chungcheongnam-do 32588 (KR)**

(72) Inventors:
• **KIM, Sang Ki**
**Gwangju 61437 (KR)**
• **RHEE, Joon Haeng**
**Gwangju 61903 (KR)**
• **LEE, Je Jung**
**Gwangju 61101 (KR)**

(74) Representative: **Marks & Clerk LLP**
**15 Fetter Lane**
**London EC4A 1BW (GB)**

Remarks:
The complete document including Reference
Table(s) and the Sequence Listing(s) can be
downloaded from the EPO website

(54) **COMPOSITION CONTAINING FEEDER CELL FOR PROLIFERATING NATURAL KILLER CELL**

(57) The present invention relates to a composition for proliferating natural killer cells, comprising feeder cells and a method for proliferating natural killer cells. Specifically, the present invention relates to a method for proliferating natural killer cells by using feeder cells that are transformed to express at least one gene selected from the group consisting of B7H6, CD137L, IL-15, and IL-15Rα.

EP 4 349 969 A1

**Description**

**Technical Field**

The present invention relates to a composition for proliferating natural killer cells, comprising feeder cells and a method for proliferating natural killer cells. Specifically, the present invention relates to a method for proliferating natural killer cells by using feeder cells that are transformed to express at least one gene selected from the group consisting of B7H6, CD137L, IL-15, and IL-15Rα.

**Background Art**

Natural killer cell (NK cell) is a representative cell responsible for innate immunity and is a type of lymphocyte that possesses large granules and is cytotoxic to various types of tumor cells and virus-infected cells. NK cells make up 5-10% of human peripheral blood lymphocytes, and unlike T cells, they mature in the liver or bone marrow. In general, immune cells eliminate infected cells by detecting infected cells through major histocompatibility complex (MHC) proteins presented on their surface and secreting various cytokines and chemicals that induce apoptosis. However, NK cells can induce an immediate immune response because they can recognize and eliminate abnormal cells even without the major histocompatibility complex bound to the antigen.

Defects in the function of NK cells have been reported in many diseases, and it is known that most NK cells are inactivated, especially in patients with cancer. Some researchers conducted research on methods to proliferate and activate NK cells in vitro, and demonstrated that NK cells amplified in vitro showed excellent cytotoxicity when reacted against various carcinomas.

Therefore, methods for proliferation of NK cells are being studied, and research is being conducted using feeder cells as one of the efficient methods for selective amplification of NK cells. However, the clear mechanism of how to amplify NK cells is unknown.

Allogeneic or autologous peripheral blood mononuclear cells (PBMC) and cancer cell lines are used as feeder cells for the amplification of NK cells. As cancer cell lines, HFWT, a Wilms tumor-derived cell line, EBV-LCL (EBV transformed lymphoblastoid cells), and the like are known.

The present inventors discovered that feeder cells expressing B7H6 have an excellent NK cell proliferation effect, and also discovered that, in addition to previously known feeder cells, ARH77 cells are excellent feeder cells and have an NK cell proliferation effect. Based on the above, the present inventors completed the present invention.

**Prior Art Document**

**Non-Patent Document**

(Non-Patent Document 1) Hiroyuki Fujisaki, Harumi Kakuda, Noriko Shimasaki, Chihaya Imai, Jing Ma, Timothy Lockey, et al. (2009) EXPANSION OF HIGHLY CYTOTOXIC HUMAN NATURAL KILLER CELLS FOR CANCER CELL THERAPY Cancer Res. Author manuscript. 2009 May 1; 69(9): 4010-4017
(Non-Patent Document 2) Denman CJ, Senyukov VV, Somanchi SS, Phatarpekar PV, Kopp LM, et al. (2012) Membrane-Bound IL-21 Promotes Sustained Ex Vivo Proliferation of Human Natural Killer Cells. PLoS ONE 7(1): e30264

**Detailed Description of Invention**

**Technical Problem**

An object of the present invention is to provide a composition for proliferating natural killer cells, comprising feeder cells that are transformed to express at least one gene selected from the group consisting of B7H6, CD137L, IL-15, and IL-15Rα.

In addition, another object of the present invention is to provide a feeder cell line that is transformed to express at least one gene selected from the group consisting of B7H6, CD137L, IL-15, and IL-15Rα and a method for proliferating natural killer cells using the same.

**Solution to Problem**

The present invention provides a composition for proliferating natural killer cells, comprising feeder cells that are transformed to express B7H6 gene.

The present invention provides a composition for proliferating natural killer cells, comprising feeder cells that are transformed to express at least one gene selected from the group consisting of B7H6, CD137L, IL-15, and IL-15R$\alpha$.

The composition of the present invention may comprise feeder cells that express B7H6, and the feeder cells may further express at least one selected from the group consisting of CD137L, IL-15, and IL-15R$\alpha$.

Preferably, the composition of the present invention may comprise feeder cells that express B7H6, CD137L, and IL-15.

More preferably, the composition of the present invention may comprise feeder cells that express B7H6, CD137L, IL-15, and IL-15R$\alpha$.

The feeder cells of the present invention may be ARH77 or K562 cells, and preferably the feeder cells may be ARH77 cells.

The present invention provides a feeder cell line that expresses B7H6.

The feeder cells of the present invention line may further express at least one selected from the group consisting of CD137L, IL-15, and IL-15R$\alpha$.

The feeder cell line of the present invention may be ARH77 or K562 cell line.

In addition, the present invention provides a method of producing feeder cells that express B7H6, comprising the steps of:

(1) transforming the B7H6 gene with a virus expression vector to produce a recombinant virus; and
(2) adding the recombinant virus to the feeder cells and culturing them.

The transgene of the present invention may further include at least one gene selected from the group consisting of CD137L, IL-15, and IL-15R$\alpha$.

The virus of the present invention may be a lentivirus or a retrovirus.

The feeder cells of the present invention may be ARH77 or K562 cells.

In addition, the present invention provides a method for proliferating natural killer cells, comprising the step of culturing feeder cells that express B7H6 and peripheral blood mononuclear cells.

The feeder cells of the present invention may further express at least one selected from the group consisting of CD137L, IL-15, and IL-15R$\alpha$.

The feeder cells of the present invention may be ARH77 or K562 cells.

The culture solution of the present invention may comprise at least one selected from the group consisting of penicillin, streptomycin, glutamine, gentamicin, fetal bovine serum, human serum, mercaptoethanol, ethanolamine, ascorbic acid, and sodium selenite. Preferably, the culture solution of the present invention may comprise at least one selected from the group consisting of glutamine, gentamicin, human serum, mercaptoethanol, ethanolamine, ascorbic acid, and sodium selenite.

The proliferation method of the present invention may further comprise the step of adding at least one selected from the group consisting of recombinant human interleukin-2, recombinant human interleukin-21, and recombinant human interleukin-15.

Preferably, the method may further comprise the steps of (a) adding recombinant human interleukin-2 and recombinant human interleukin-21, and (b) adding recombinant human interleukin-2 and recombinant human interleukin-15.

In addition, the present invention may provide a natural killer cell line produced by the proliferation method of the present invention.

**Effects of Invention**

The feeder cells that express at least one gene selected from the group consisting of B7H6, CD137L, IL-15, and IL-15R$\alpha$ according to the present invention have the effect of obtaining NK cells with higher levels of expansion rate, purity, and cytotoxicity when the feeder cells according to the present invention are used to proliferate natural killer cells (NK cells) compared to the method using the previously known feeder cells. Therefore, the feeder cells according to the present invention can be very useful in various immune cell therapies using NK cells, etc.

**Brief Description of Drawings**

Fig. 1 shows the structure of a lentiviral vector.

Figs. 2a and 2b show the sequence of introducing each gene into ARH77 cells and the process of selecting cells expressing the gene.

Figs. 3 and 4 show the expansion rate of NK cells obtained through K562 cells, B7H6 expressing K562 (K562-B7H6) cells, and CD137L expressing K562 (K562-CD137L) cells.

Figs. 5 and 6 show the purity of NK cells obtained through K562 cells, B7H6 expressing K562 (K562-B7H6) cells, and CD137L expressing K562 (K562-CD137L) cells.

Figs. 7 and 8 show the cytotoxicity of NK cells obtained through K562 cells, B7H6 expressing K562 (K562-B7H6) cells, and CD137L expressing K562 (K562-CD137L) cells.

Fig. 9 shows the expansion rate of NK cells obtained through ARH77 cells and IL15Rα expressing ARH77 (ARH77-IL15Rα) cells.

Fig. 10 shows the purity of NK cells obtained through ARH77 cells and IL15Rα expressing ARH77 (ARH77-IL15Rα) cells.

Fig. 11 shows the cytotoxicity of NK cells obtained through ARH77 cells and IL15Rα expressing ARH77 (ARH77-IL15Rα) cells.

Fig. 12 shows the expansion rate of NK cells obtained through ARH77 cells and K562 cells.

Figs. 13 and 14 show the purity of NK cells obtained through ARH77 cells and K562 cells.

Fig. 15 shows the cytotoxicity of NK cells obtained through ARH77 cells and K562 cells.

Figs. 16 to 22 show the mean fluorescence intensity (MFI) of expression levels of the receptor that plays an important role in NK cell activation in ARH77 cells and K562 cells.

Fig. 23 shows the expansion rate of NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells.

Fig. 24 shows the purity of NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells.

Fig. 25 shows the cytotoxicity of NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells.

**Best Mode for Carrying out the Invention**

Hereinafter, with reference to the accompanying drawings, embodiments and examples of the present disclosure will be described in detail so that those of ordinary skill in the art to which the present invention belongs can easily practice the present invention. However, the present disclosure may be implemented in various forms and is not limited to the embodiments and examples described herein.

Throughout the present specification, when a certain part "includes" a certain component, it means that other components may be further included, rather than excluding other components, unless otherwise stated.

The present invention relates to a composition for proliferating natural killer cells, comprising feeder cells that are transformed to express B7H6 gene and a method for proliferating natural killer cells.

The present invention relates to a composition for proliferating natural killer cells, comprising feeder cells that are transformed to express at least one gene selected from the group consisting of B7H6, CD137L, IL-15, and IL-15Rα and a method for proliferating natural killer cells.

As used herein, the term "B7H6" refers to the ligand of the natural killer cell receptor NKp30.

The B7H6 gene according to the present invention may be composed of the nucleotide sequence of SEQ ID NO: 1, and homologs of the nucleotide sequence are included within the scope of the present invention. Specifically, the gene may comprise a nucleotide sequence having sequence homology of at least 70%, preferably at least 80%, more preferably at least 90%, and most preferably at least 95% with the nucleotide sequence of SEQ ID NO: 1.

As used herein, the term "CD137L" refers to the ligand of CD137.

The CD137L gene according to the present invention may be composed of the nucleotide sequence of SEQ ID NO: 2, and homologs of the nucleotide sequence are included within the scope of the present invention. Specifically, the gene may comprise a nucleotide sequence having sequence homology of at least 70%, preferably at least 80%, more preferably at least 90%, and most preferably at least 95% with the nucleotide sequence of SEQ ID NO: 2.

As used herein, the term "IL-15" refers to interleukin-15, which is a cytokine produced in epithelial cells.

The IL-15 gene according to the present invention may be composed of the nucleotide sequence of SEQ ID NO: 3, and homologs of the nucleotide sequence are included within the scope of the present invention. Specifically, the gene may comprise a nucleotide sequence having sequence homology of at least 70%, preferably at least 80%, more preferably at least 90%, and most preferably at least 95% with the nucleotide sequence of SEQ ID NO: 3.

As used herein, the term "IL-15Rα" refers to interleukin 15 receptor α-chain.

The IL-15Rα gene according to the present invention may be composed of the nucleotide sequence of SEQ ID NO: 4, and homologs of the nucleotide sequence are included within the scope of the present invention. Specifically, the gene may comprise a nucleotide sequence having sequence homology of at least 70%, preferably at least 80%, more preferably at least 90%, and most preferably at least 95% with the nucleotide sequence of SEQ ID NO: 4.

As used herein, the term "feeder cell (supporting cell)" refers to a cell that does not have the ability to divide and proliferate, but is metabolically active and produces various metabolites to help the proliferation of target NK cells. The feeder cells that can be used in the present invention include animal cell lines into which genes have been introduced, peripheral blood leukocytes (PBL) treated with various cytokines or compounds, one's own or another person's peripheral blood leukocytes, T cells, B cells, or monocytes, etc. Preferably, animal cell lines into which genes have been introduced can be used. More preferably, the feeder cells may be ARH77 cells, but are not limited thereto. Other feeder cells known to be commonly available in the technical field to which the present invention belongs can also be used without limitation as long as they meet the purpose of the present invention.

As used herein, the term "expression vector" is a vector capable of expressing a target protein or target RNA in a suitable host cell, and refers to a gene construct comprising essential regulatory elements operably linked to express the gene insert.

As used herein, the term "operably linked" refers to a functional linkage between a nucleic acid expression control sequence and a nucleic acid sequence encoding a desired protein or RNA to perform a general function. For example, a promoter and a nucleic acid sequence encoding a protein or RNA can be operably linked to affect expression of the encoding nucleic acid sequence. Operably linking with a recombinant expression vector can be achieved using genetic recombination techniques well known in the art, and site-specific DNA cleavage and ligation can be achieved using enzymes commonly known in the art.

Expression vectors that can be used in the present invention include, but are not limited to, plasmid vectors, cosmid vectors, bacteriophage vectors, viral vectors, and the like. Preferably, the vector may be a viral vector, and more preferably a lentiviral or retroviral vector, but is not limited thereto. In addition, suitable expression vectors can be prepared in various ways depending on the purpose, including a signal sequence or a leader sequence for membrane targeting or secretion, in addition to an expression control sequence such as a promoter, an operator, an initiation codon, a termination codon, a polyadenylation signal, and an enhancer. The promoter of an expression vector can be constitutive or inducible. In addition, the expression vector includes a selection marker for selecting host cells containing the vector, and, in the case of an expression vector capable of replication, includes an origin of replication.

As used herein, the term "peripheral blood mononuclear cell (PBMC)" refers to a cell with a spherical nucleus present in the blood. The peripheral blood mononuclear cells include immune cells such as B cells, T cells, macrophages, dendritic cells, and natural killer cells (NK cells).

"IL-2 (interleukin-2)" as used herein has been reported to have the function of enhancing the proliferation and activation of mature NK cells. There are reports that the number of NK cells is significantly reduced in humans and mice deficient in IL-2. On the other hand, there are also studies showing that IL-2 and IL-2Ra deficiency indirectly affects the number and activation of NK cells. The IL-2R chain is known to be involved in forming the IL-15 receptor.

Regarding "IL-15 (interleukin-15)" as used herein, it was found that NK cells were not found in mice deficient in IL-15 or IL-15Rα. In addition, it was found that NK cells were deficient in mice deficient in transcription factor interferon (IFN)-regulatory factor 1 that is required for IL-15 production. Accordingly, it is known that IL-15 is involved in NK cell differentiation and that IL-15 directly promotes the growth and differentiation of NK cells through the IL-15 receptor expressed on NK cells.

"IL-21 (interleukin-21)" as used herein is known to increase the telomere length of NK cells by stimulating transcription-3 (STAT-3) and to play an important role in the activation, growth, and amplification of NK cells. In particular, it has been reported that IL-21 helps the growth of NK cells in human CD34+ hematopoietic stem cells through combination with IL-15. It was confirmed that when NK cells are amplified through membrane-bound (mb) IL-21, the expression and cytotoxicity of interferon gamma (IFN-γ) are increased. In addition, it has been reported that a single initial injection of IL-21 is more effective in amplifying NK cells than continuous injection of IL-21 into the culture solution.

Hereinafter, the present invention will be described in more detail through the examples, but the following examples are for illustrative purposes only and are not intended to limit the scope of the present invention.

**[Preparation Example]**

**Production of B7H6, CD137L, IL-15, and IL-15Rα expressing ARH77 (ARH77-B7H6-CD137L-IL15-IL15Rα) cells**

The genes for B7H6, CD137L, IL-15, and IL-15Rα were cloned, and then the cDNA of each gene was transferred to the pLVX-EF1α-IRES-Puro (Takara, Japan) plasmid, a lentivirus expression vector, to produce a lentivirus.

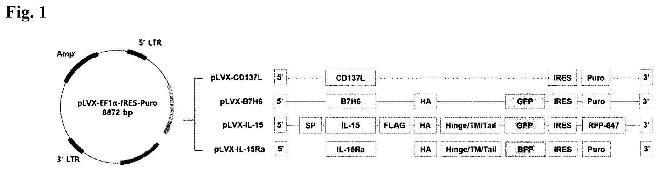

As shown in Fig. 1, the CD137L gene, B7H6 and EGFP genes, IL-15 and RFP-647 genes, and IL-15Rα and BFP genes were inserted into the multiple cloning site of each pLVX-EF1α-IRES-Puro plasmid. The lentiviral plasmid (10 μg) into which each gene was introduced was transfected into HEK 293T cells together with Lenti-X™ Packaging Single shots (Takara) and cultured in DMEM culture solution containing 10% FBS for 48 to 72 hours. Thus, a recombinant lentivirus was produced.

In order to transduce each gene into feeder cells, ARH77 cells and K562 cells were dispensed at $2 \times 10^5$ cells per well in a 24 well plate, and then the recombinant lentivirus was added to each well along with polybrene (8 μg/mL). The mixture was centrifuged at 1,500g for 2 hours at 25 °C and then cultured for 48 hours in an incubator at 37 °C and 5% $CO_2$ conditions. Thereafter, the culture solution was removed, and culture was maintained while replacing with fresh culture solution (2 mL/well). One week after transduction, all cells were individually recovered, and then only fluorescence-positive cells were isolated using a cell sorting device, BD FACSAria™ III, and these cells were continued to be cultured in RPMI1640 culture solution containing 10% FBS.

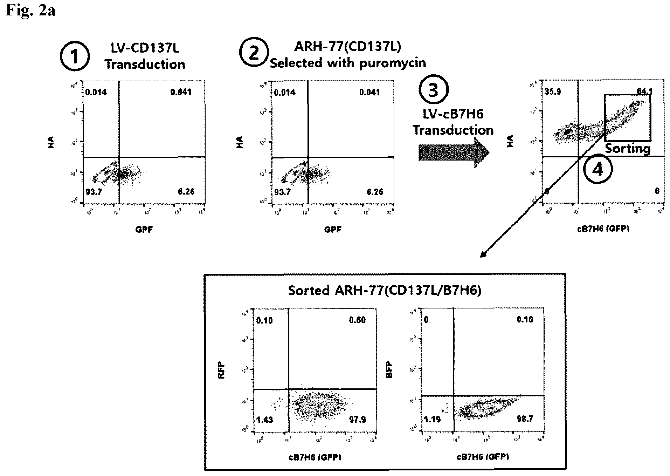

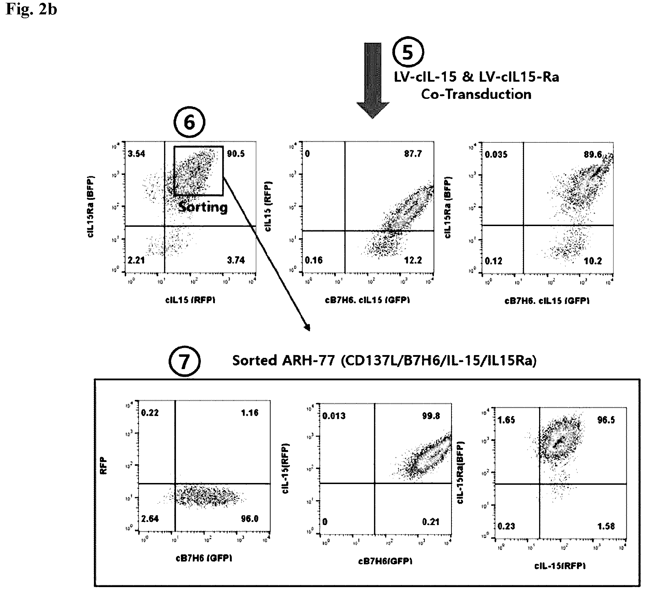

In the same manner as above, each gene was introduced into ARH77 cells and K562 cells in the order shown in Fig. 2, and the cells were subjected to a selection process to produce ARH77-B7H6-CD137L-IL15-IL15Rα and K562-B7H6-CD137L-IL15-IL15Rα cells.

The nucleotide sequences of B7H6, CD137L, IL-15, and IL-15Rα are listed in Table 1 below.

[Table 1]

| Gene | SEQ ID NO | Nucleotide sequence |
|------|-----------|---------------------|
| B7H6 | SEQ ID NO: 1 | ATGGCGCACGGGCCGCCGCTCCTGCTCCTGCCCGCGCTCCTG CTCCTGCCGGCGCTCCTGGTCCTGGTCCTGTGGTTCGGGACG GCCAGCTCAGGTCTCCTGCAAGTGGAGATGGCAGGGAAGAC TCAGAAGGTGCTCCTAAATGATACTGCCACCATTGTCTGCAA AATCCATGGTTACCACCATCTGGACATCACAGTAATGGGTATT ACCTGGTATTGGAAGCATCAGGCCTCTGCAACGGAAGTTAAA CTGTTTGAGTTTTTTGGTGACCATCAAATGATATTACGATCTG GAGCCTACGTGTCTCAAAGGAGGCTGCAAAGGGGGGATGCC TCCCTCCAGCTGCCAGGCGTCCAGCTGAAGGAAGCAGGAGA GTACCGATGTGAGGTGGTGGTCACCCCTTACAAAGCAGTGGG AACAGTCAACCTGGAGGTTGTGGCTTACCCCGTCAGCAGCTT GTCTCCAGAGCAAGCCATGGTGAAAGAAAATGAAGAACAAC TTATTTTGTGTATGGCAAGTAGGTTCTACCCCAAGAACATTAC CATAACATGGAAAAAGTGGACCCCAAAGGATCCCCGGTATCT GGAGGTTTCTGAGGGCATCATTACTAATGGTACCACCGAAGAT GAGGATGGCATGTTTAGTGTCACTAGTTACTTGATGGTGAAGC CCTCTTTGGAAGACAATATGACTGTCTATCAGTGTGTGGTATG GCATGAATCCTTGCCAATCTCCCAGAGCCGCAACTTCACCTT GATT |

(continued)

| Gene | SEQ ID NO | Nucleotide sequence |
|---|---|---|
| CD137L | SEQ ID NO: 2 | ATGCGCCCCGCAGCGACGCCGCCCCGGACCCCGAGGCCCCGCGGCCGCCCGCGCCCCCGGCCGCACCTGCAGCCCGCTGCCCTGGGCGCTGAGCGCCGCGATGCTGCTGCTCGTCGGCACCTGCGCCGCCTGCGCGCTCCGCGCCTGGGTGGTCCCCGGGCCCCGGCCCCCGCGCTCCCCGCGCTCCCCGCGCTCCCCGCGCCCCTGCCGGACGCCGGCGCCCGCCTCCCCGACTCCCCGCAGGCCGTGTTCGCGCAGCTGGTGGCCCGAGATGTACAGCTGAAGGAAGGACCCCTGCGCTGGTACAGTGACCCGGGCCTGGCAGGTGTATTCCTGGGGCCGGGCCTGAGTTATGACCAGCACACTCGGGAGCTGATGGTGGTGGAACCCGGGCTCTACTATGTTTTCTTGCACCTGAAGCTGCAGCGGGTAATGTCCAGCACGGGCTCCGGCTCTGTCTCTGCTGCCCTGCACCTGCAGCCACTTGGCACCGAGGCTGCAGCCCTGGACCTGACCTTGGACCTGCCTCCACCATCCTCGGAGGCCCGTGACTCAGCAGCTGGTTTCCAGGGCAGCCTGCTGCACCTGGACGCAGGCCAGCGCCTCCGTGTTCACTTGCGAGCTGAGGCAGGGGCCCACCCTGCCTGGCAGCTGGCACAAGGTGCCACGATCTTGGGCCTCTTCAGAGTGGCCACCAAAGTCCCCACTGGACTCCCTCGTCATGGCCCATGGACACGGGGCCTGGGTCCCCGCCCCTGGATGGAGAATAA |
| IL-15 | SEQ ID NO: 3 | aattggcaggacgtgatacttgatttggaaaaaattgacaatcttattcaatctatacatatggataccactctgtatactgaaagtgatgtgcatcccagttgcaaagtaaccgcgatgaagtgctttctcctggagttaggtgttatctcgctcgagtccggcagtcatcccattaaggaagcagtagagaacctcatcatcctcgcaaacagtgatctgtcttcgaaggggaatataactgaaacgggatgcaaagaatgtgaagaactggaggaaaagagtattaaggagtttttgcagagtttcgtgcatatcgtacaaatgttcatcaactcctct |
| IL-15Rα | SEQ ID NO: 4 | atggcgcggacggggcggacggggccgccgctccggggccgccggctccccgcgctccccgcgctccccgcgctgctgctgctgctgccgctccggcccgcgacgccgggcatcacatgccctcctcccacatctgtcaaacacgcagatatccaggtcaagagttacagcgtgagctccaaggaacggtacacttgtaactctggcttcaagcggaaagccgggacatccagcctgactgagtgtgtgctcaacaagaccaccaacactgcccactggacaatccccagtctcaagtgcatcagagacccctccctgactcacctaaggccatcctcctcagacgtgccggcaggggtgaccccagagccagagagcacctcccttctggaaaaagagccaactttcacttccaagtcagacaccaaagtggccacaaagccaactattgtgccaggctccaggccgatgccgtcaaagcctcctgctacaggaaccacagggctgatcagtaatgaacctcctcccaagccccatctcagacaacagcaaaggccttggaacacactccctctgcctcccaggagacgcca |

**[Example 1]**

**NK cell activation effect by B7H6 or CD137L expressing feeder cells**

**1-1. Evaluation of NK cell expansion rate**

In order to confirm the NK cell activation effect by B7H6 or CD137L expression, the expansion rate of NK cells was evaluated using K562 cells, B7H6 expressing K562 (K562-B7H6) cells, and CD137L expressing K562 (K562-CD137L) cells.

PBMCs were isolated from the peripheral blood of healthy blood donors using density gradient centrifugation and washed twice with PBS, and then $3\times10^6$ PBMCs were co-cultured with $5 \times 10^5$ respective feeder cells (K562 cells, K562-B7H6 cells, or K562-CD137L cells) irradiated with 100 Gy of gamma radiation in a 24 well plate with 2 mL/well of culture solution in an incubator at 37 °C and 5% $CO_2$ conditions.

As a culture solution, RPMI1640 culture solution containing 10% fetal bovine serum (FBS), 100 U.mL penicillin, 100 $\mu$g/mL streptomycin, and 4 mmol/L L-glutamine was used. For one week from the start of culture, 10 U/mL recombinant human interleukin (rhIL)-2 and 5 ng/mL rhIL-21 were added to the culture solution, and after 1 week of culture, 100 U/mL rhIL-2 and 5 ng/mL rhIL-15 were added to the culture solution, and cultured for 28 days. The culture solution was replaced with new culture solution every 1 to 2 days.

On days 14, 21, and 28 of culture, the cultured cells were recovered, and the ratio (purity) of NK cells (CD3-, CD56+ cells) in the recovered cells was evaluated by flow cytometry. The total number of NK cells was calculated by multiplying the total number of viable cells among the recovered cells by the ratio of NK cells. The total number of NK cells in PBMCs prior to culture was calculated in the same manner. The expansion rate of NK cells was calculated by dividing the total number of NK cells on days 14, 21, and 28 of culture by the number of NK cells prior to culture, respectively.

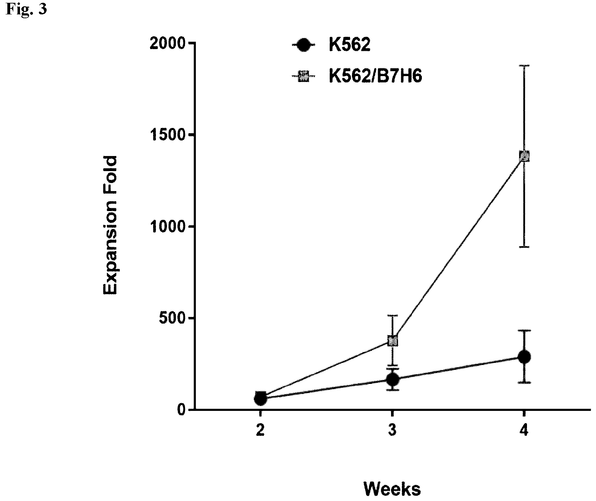

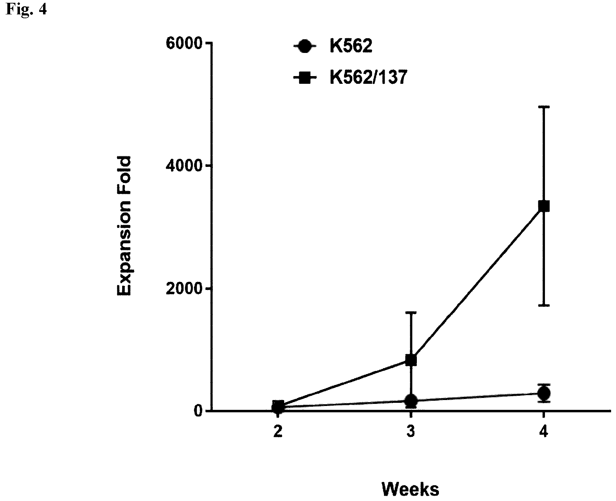

The experimental results are shown in Tables 2 and 3 below and Figs. 3 and 4.

[Table 2]

| Fold | 2 weeks | | 3 weeks | | 4 weeks | |
|---|---|---|---|---|---|---|
| | Mean | SD | Mean | SD | Mean | SD |
| K562 | 61 | 30 | 167 | 58 | 291 | 141 |
| K562/B7H6 | 72 | 23 | 378 | 136 | 1383 | 495 |

[Table 3]

| Fold | 2 weeks | | 3 weeks | | 4 weeks | |
|---|---|---|---|---|---|---|
| | Mean | SD | Mean | SD | Mean | SD |
| K562 | 61 | 30 | 167 | 58 | 291 | 141 |
| K562/CD137L | 82 | 13 | 835 | 778 | 3344 | 1621 |

As shown in Table 2 above and Fig. 3, NK cells obtained through K562 cells exhibited an expansion rate of 291 times after 4 weeks of culture, while NK cells obtained through B7H6 expressing K562 (K562-B7H6) cells exhibited an expansion rate of 1,383 times after 4 weeks of culture.

In addition, as shown in Table 3 above and Fig. 4, NK cells obtained through CD137L expressing K562 (K562-CD137L) cells exhibited an expansion rate of 3,344 times after 4 weeks of culture.

Therefore, B7H6 expressing K562 (K562-B7H6) cells exhibited an expansion rate of 4 times or more compared to K562 cells, and CD137L expressing K562 (K562-CD137L) cells exhibited an expansion rate of 11 times or more, and thus, it can be seen that the cells have a more excellent NK cell expansion effect.

**1-2. Evaluation of NK cell purity**

In order to confirm the NK cell activation effect by B7H6 or CD137L expression, the purity of NK cells was evaluated using K562 cells, B7H6 expressing K562 (K562-B7H6) cells, and CD137L expressing K562 (K562-CD137L) cells.

In the same manner as in Example 1-1 above, PBMCs were isolated and co-cultured with each feeder cell irradiated with radiation. PBMC prior to culture and each cell recovered on days 14, 21, and 28 of culture were stained with fluorescent-conjugated human CD3 and CD56 monoclonal antibodies, and then the purity of NK cells was evaluated by flow cytometry. The ratio of CD3- CD56+ NK cells in each cell was evaluated as the purity of NK cells.

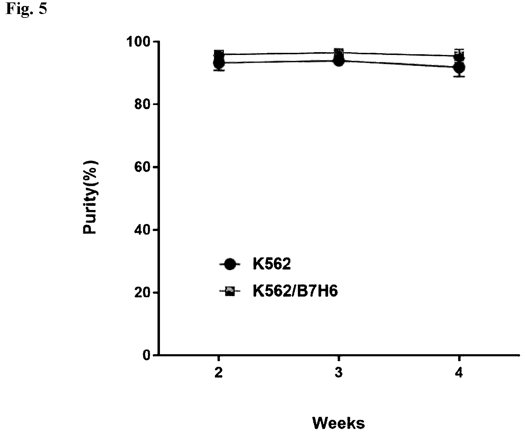

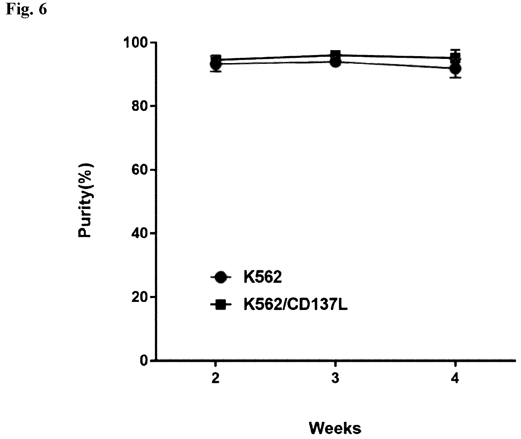

The experimental results are shown in Tables 4 and 5 below and Figs. 5 and 6.

[Table 4]

| Purity | 2 weeks | | 3 weeks | | 4 weeks | |
|---|---|---|---|---|---|---|
| | Mean | SD | Mean | SD | Mean | SD |
| K562 | 93 | 2 | 94 | 1 | 92 | 3 |
| K562/B7H6 | 96 | 1 | 97 | 1 | 96 | 2 |

[Table 5]

| Purity | 2 weeks | | 3 weeks | | 4 weeks | |
|---|---|---|---|---|---|---|
| | Mean | SD | Mean | SD | Mean | SD |
| K562 | 93 | 2 | 94 | 1 | 92 | 3 |
| K562/CD137L | 95 | 1 | 96 | 1 | 95 | 3 |

As shown in Tables 4 and 5 above and Figs. 5 and 6, NK cells obtained through K562 cells, B7H6 expressing K562 (K562-B7H6) cells, and CD137L expressing K562 (K562-CD137L) cells all exhibited a purity of 90% or more.

Therefore, it can be seen that both B7H6 expressing K562 (K562-B7H6) cells and CD137L expressing K562 (K562-CD137L) cells can proliferate NK cells with high purity.

### 1-3. Evaluation of NK cell cytotoxicity

In order to confirm the NK cell activation effect by B7H6 or CD137L expression, the cytotoxicity capability against cancer cells of NK cells that were proliferated using K562 cells, B7H6 expressing K562 (K562-B7H6) cells, and CD137L expressing K562 (K562-CD137L) cells was evaluated.

The killing ability ofNK cells proliferated in the same manner as in Example 1-1 above against K562 cells was measured by a highly sensitive colorimetric assay using WST-8 (Biotool, USA). K562 cells ($1 \times 10^5$ cells/100 $\mu$L of culture solution), which are cancer cells targeted by NK cells, were dispensed in a 96 well plate, and then $2.5 \times 10^4$ proliferated NK cells were added and mixed (effector: target ratio = 0.25:1). Thereafter, the cells were centrifuged at 1,500 rpm for 3 minutes and cultured in an incubator at 37 °C and 5% $CO_2$ conditions. After 3 hours of culture, 10 $\mu$L of WST-8 was added to each well and then cultured for another hour. Thereafter, the absorbance (A450) was measured using SpectraMax (Molecular Devices, USA) at a wavelength of 450 nm, and the ratio (%) of cancer cells killed by NK cells was calculated using the following calculation formula.

Toxicity (Cytotoxicity, %) = 100% - 100 X [A450 of effector cell treated target cells - A450 of effector cells] / [A450 of target cells - A450 of target cells without WST-8]

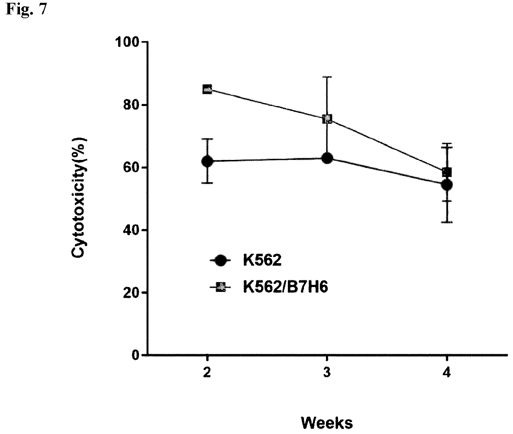

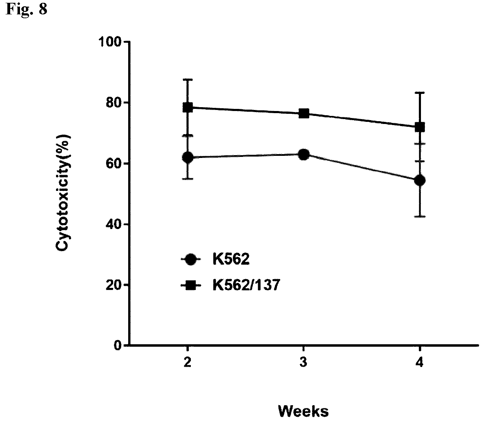

The experimental results are shown in Tables 6 and 7 below and Figs. 7 and 8.

[Table 6]

| Toxicity (%) | 2 weeks | | 3 weeks | | 4 weeks | |
|---|---|---|---|---|---|---|
| | Mean | SD | Mean | SD | Mean | SD |
| K562 | 62 | 7 | 63 | 0 | 55 | 12 |
| K562/B7H6 | 85 | 1 | 76 | 13 | 59 | 9 |

[Table 7]

| Toxicity (%) | 2 weeks | | 3 weeks | | 4 weeks | |
|---|---|---|---|---|---|---|
| | Mean | SD | Mean | SD | Mean | SD |
| K562 | 62 | 7 | 63 | 0 | 55 | 12 |
| K562/CD137L | 78.5 | 9.192388 | 76.5 | 0.707107 | 72 | 11.31371 |

As shown in Tables 6 and 7 above and Figs. 7 and 8, NK cells obtained through B7H6 expressing K562 (K562-B7H6) cells and CD137L expressing K562 (K562-CD137L) cells exhibited a higher cytotoxicity than NK cells obtained through K562 cells.

Therefore, it can be seen that B7H6 expressing K562 (K562-B7H6) cells and CD137L expressing K562 (K562-CD137L) cells can proliferate NK cells exhibiting a higher level of cytotoxicity.

[Example 2]

**NK cell activation effect by IL15Rα expressing feeder cells**

**2-1. Evaluation of NK cell expansion rate**

In order to confirm the NK cell activation effect by IL15Rα expression, the expansion rate of NK cells was evaluated using ARH77 cells and IL15Rα expressing ARH77 (ARH77-IL15Rα) cells.

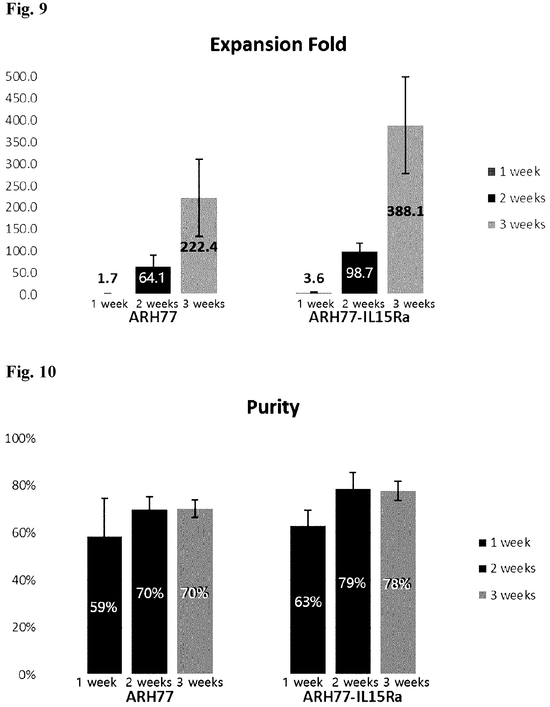

The experiment was conducted in the same manner as in Example 1-1 above, and only IL-2 and IL-15 were added to the culture solution, and the cells were cultured and evaluated for 3 weeks. The experimental results are shown in Table 8 below and Fig. 9.

[Table 8]

| Fold | 1 week | | 2 weeks | | 3 weeks | |
|---|---|---|---|---|---|---|
| | Mean | SD | Mean | SD | Mean | SD |
| ARH77 | 1.7 | 1.01 | 64.1 | 26.2 | 222.4 | 88.3 |
| ARH77/IL15Rα | 3.6 | 1.31 | 98.7 | 18.8 | 388.1 | 111.1 |

As shown in Table 8 above and Fig. 9, NK cells obtained through ARH77 cells exhibited an expansion rate of 222 times after 3 weeks of culture, while NK cells obtained through IL15Rα expressing ARH77 (ARH77-IL15Rα) cells exhibited an expansion rate of 388 times after 3 weeks of culture.

Therefore, IL15Rα expressing ARH77 (ARH77-IL15Rα) cells exhibited an expansion rate of 1.5 times or more compared to ARH77 cells, and thus, it can be seen that the cells have a more excellent NK cell expansion effect.

**2-2. Evaluation of NK cell purity**

In order to confirm the NK cell activation effect by IL15Rα expression, the purity of NK cells was evaluated using ARH77 cells and IL15Rα expressing ARH77 (ARH77-IL15Rα) cells.

The experiment was conducted in the same manner as in Example 1-2 above and evaluated for 3 weeks. The experimental results are shown in Table 9 below and Fig. 10.

[Table 9]

| Purity | 1 week | | 2 weeks | | 3 weeks | |
|---|---|---|---|---|---|---|
| | Mean | SD | Mean | SD | Mean | SD |
| ARH77 | 59 | 0.16 | 70 | 0.05 | 70 | 0.04 |
| ARH77/IL15Rα | 63 | 0.07 | 79 | 0.07 | 78 | 0.04 |

As shown in Table 9 above and Fig. 10, NK cells obtained through ARH77 cells exhibited a purity of 59 to 70%, while NK cells obtained through IL15Rα expressing ARH77 (ARH77-IL15Rα) cells exhibited a purity of 63 to 79%, which is higher than that of the above cells.

Therefore, it can be seen that NK cells obtained through IL15Rα expressing ARH77 (ARH77-IL15Rα) cells have higher purity, and IL15Rα expressing ARH77 (ARH77-IL15Rα) cells can proliferate NK cells with higher purity.

**2-3. Evaluation of NK cell cytotoxicity**

In order to confirm the NK cell activation effect by IL15Rα expression, the cytotoxicity capability against cancer cells of NK cells that were proliferated using ARH77 cells and IL15Rα expressing ARH77 (ARH77-IL15Rα) cells was evaluated.

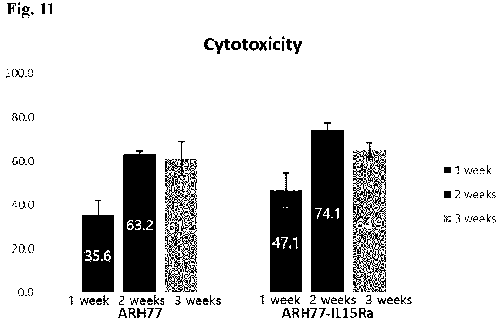

The experiment was conducted in the same manner as in Example 1-3 above and evaluated for 3 weeks. The experimental results are shown in Table 10 below and Fig. 11.

[Table 10]

| Toxicity (%) | 1 week | | 2 weeks | | 3 weeks | |
|---|---|---|---|---|---|---|
| | Mean | SD | Mean | SD | Mean | SD |
| ARH77 | 35.6 | 6.45 | 63.2 | 1.42 | 61.2 | 7.62 |
| ARH77/IL15Rα | 47.1 | 7.44 | 74.1 | 3.12 | 64.9 | 3.12 |

As shown in Table 10 above and Fig. 11, NK cells obtained through IL15Rα expressing ARH77 (ARH77-IL15Rα) cells exhibited a higher cytotoxicity than NK cells obtained through ARH77 cells.

Therefore, it can be seen that IL15Rα expressing ARH77 (ARH77-IL15Rα) cells can proliferate NK cells exhibiting a higher level of cytotoxicity.

**[Example 3]**

**NK cell stimulating effect of ARH77 cells**

**3-1. Evaluation of NK cell expansion rate**

In order to confirm the NK cell stimulating effect of ARH77 cells, the expansion rate of NK cells was evaluated using ARH77 cells and K562 cells.

In the same manner as in Example 1-1 above, PBMCs were isolated and co-cultured with ARH77 cells or K562 cells irradiated with radiation. PBMC prior to culture and each cell recovered on days 14, 21, and 28 of culture were stained with fluorescent-conjugated human CD3 and CD56 monoclonal antibodies, and then the purity of NK cells (CD3-CD56+ cells) was evaluated by flow cytometry. The total number of NK cells was calculated by multiplying the total number of viable cells among the recovered cells by the ratio of NK cells. The total number of NK cells in PBMCs prior to culture was calculated in the same manner, and the expansion rate of NK cells was calculated by dividing the total number of NK cells on days 14, 21, and 28 of culture by the number of NK cells prior to culture, respectively.

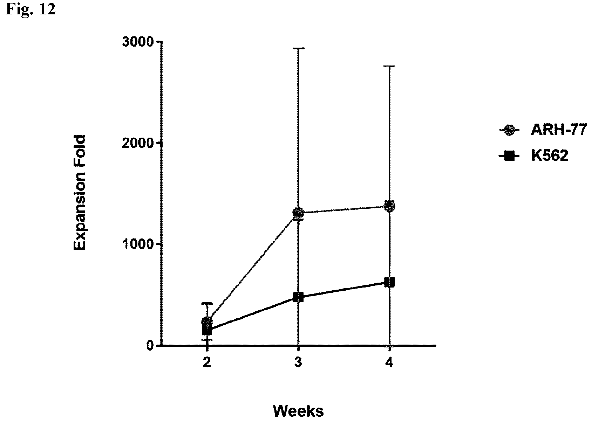

The experimental results are shown in Table 11 below and Fig. 12.

[Table 11]

| Expansion rate (fold) | 2 weeks | | 3 weeks | | 4 weeks | |
|---|---|---|---|---|---|---|
| | Mean | SD | Mean | SD | Mean | SD |
| ARH77 | 234 | 176 | 1313 | 1628 | 1376 | 1386 |
| K562 | 150 | 267 | 477 | 766 | 627 | 798 |

As shown in Table 11 above and Fig. 12, NK cells obtained through K562 cells exhibited an expansion rate of 627 times after 4 weeks of culture, while NK cells obtained through ARH77 cells exhibited an expansion rate of 1,376 times after 4 weeks of culture.

Therefore, it can be seen that ARH77 cells have a more excellent NK cell expansion effect by 2 times or more.

**3-2. Evaluation of NK cell purity**

In order to confirm the NK cell stimulating effect of ARH77 cells, the purity of NK cells obtained using ARH77 cells and K562 cells was evaluated.

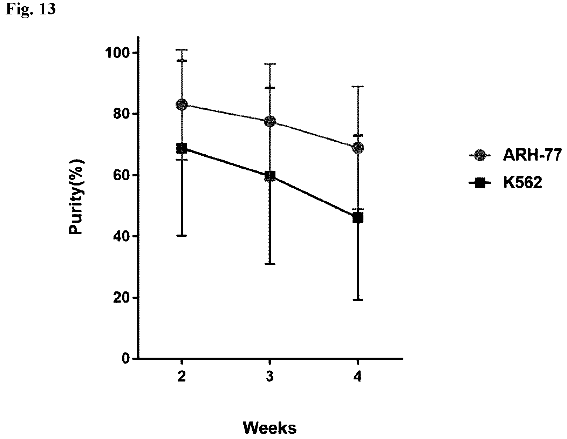

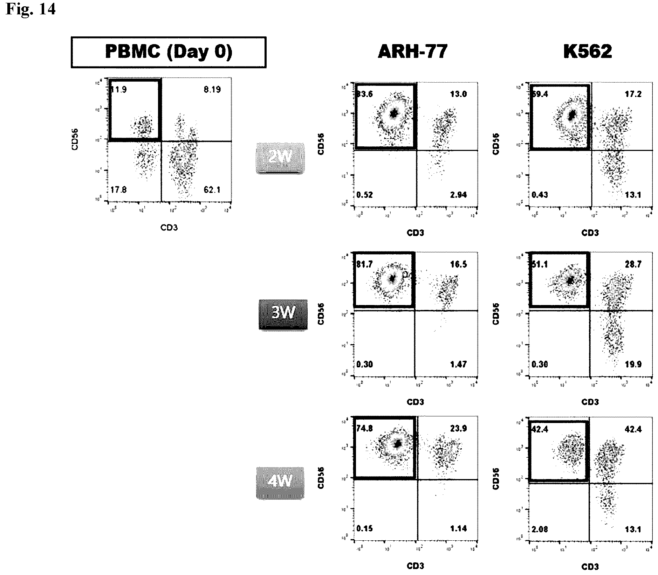

The purity was evaluated by measuring the ratio of CD3- CD56+ NK cells in the cell by flow cytometry in the same manner as in Example 1-2 above, and the experimental results are shown in Table 12 below and Figs. 13 and 14.

[Table 12]

| Purity | 2 weeks | | 3 weeks | | 4 weeks | |
|---|---|---|---|---|---|---|
| | Mean | SD | Mean | SD | Mean | SD |
| ARH77 | 83.1 | 18 | 77.6 | 19 | 69.0 | 20 |
| K562 | 68.8 | 29 | 59.7 | 29 | 46.1 | 27 |

As shown in Table 12 above and Figs. 13 and 14, NK cells obtained through K562 cells exhibited a purity of 46.1 to 68.7%, while NK cells obtained through ARH77 cells exhibited a purity of 69.0 to 83.1%, which is higher than that of the above cells.

Therefore, it can be seen that NK cells obtained through ARH77 cells have higher purity.

**3-3. Evaluation of NK cell cytotoxicity**

In order to confirm the NK cell stimulating effect of ARH77 cells, the cytotoxicity of NK cells obtained using ARH77 cells and K562 cells was evaluated.

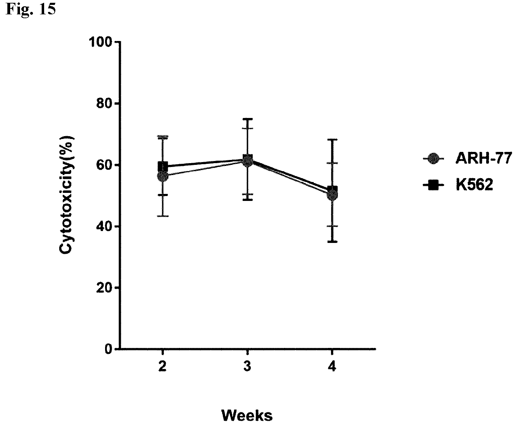

In the same manner as in Example 1-3 above, the cytotoxicity capability against cancer cells of NK cells obtained using ARH77 cells or K562 cells was measured, and the experimental results are shown in Table 13 below and Fig. 15.

[Table 13]

| Toxicity (%) | 2 weeks | | 3 weeks | | 4 weeks | |
|---|---|---|---|---|---|---|
| | Mean | SD | Mean | SD | Mean | SD |
| ARH77 | 56 | 13 | 61 | 11 | 50 | 10 |
| K562 | 60 | 9 | 62 | 13 | 52 | 17 |

As shown in Table 13 above and Fig. 15, it was confirmed that the cytotoxicity of NK cells obtained through ARH77 cells and K562 cells was significant.

Therefore, it can be seen that ARH77 cells can proliferate NK cells exhibiting a similar level of cytotoxicity to K562 cells.

**3-4. Evaluation of NK cell phenotype**

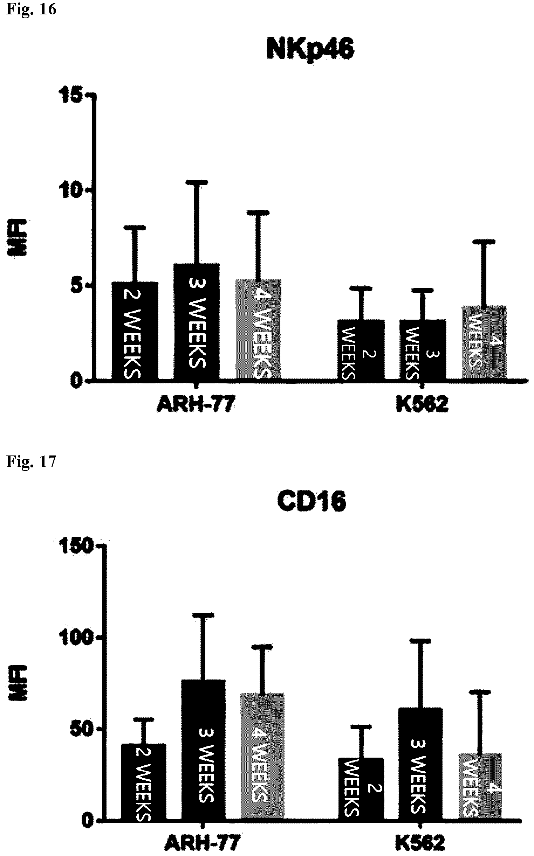

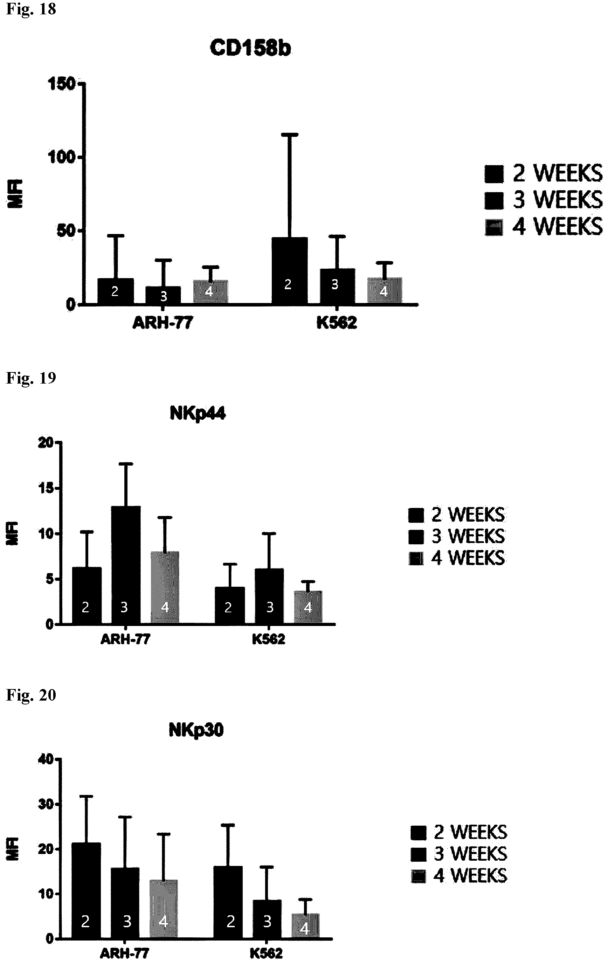

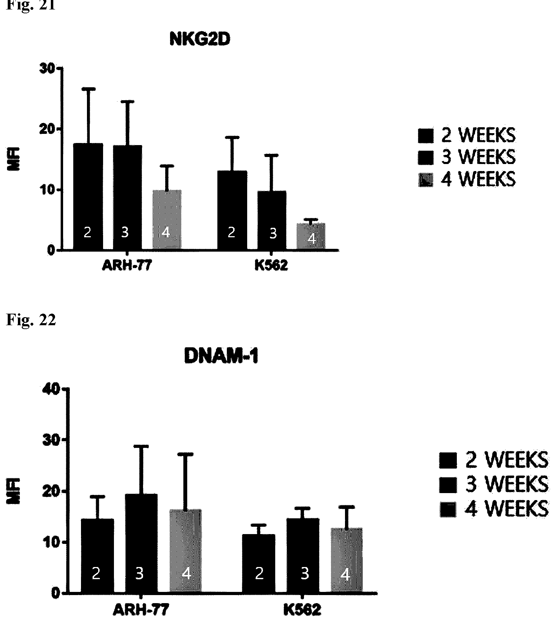

In order to confirm the NK cell stimulating effect of ARH77 cells, the receptor expression of NK cells obtained using ARH77 cells and K562 cells was evaluated, and the results are shown in Tables 14 to 20 below and Figs. 16 to 22.

[Table 14]

| NKp46 MFI | 2 weeks | | 3 weeks | | 4 weeks | |
|---|---|---|---|---|---|---|
| | Mean | SD | Mean | SD | Mean | SD |
| ARH77 | 5 | 3 | 6 | 4 | 5 | 4 |
| K562 | 3 | 2 | 3 | 2 | 4 | 3 |

[Table 15]

| CD16 MFI | 2 weeks | | 3 weeks | | 4 weeks | |
|---|---|---|---|---|---|---|
| | Mean | SD | Mean | SD | Mean | SD |
| ARH77 | 41 | 15 | 76 | 37 | 69 | 26 |
| K562 | 33 | 18 | 60 | 38 | 36 | 34 |

[Table 16]

| CD158b MFI | 2 weeks | | 3 weeks | | 4 weeks | |
|---|---|---|---|---|---|---|
| | Mean | SD | Mean | SD | Mean | SD |
| ARH77 | 17 | 30 | 11 | 19 | 16 | 10 |
| K562 | 44 | 71 | 24 | 22 | 17 | 11 |

[Table 17]

| NKp44 MFI | 2 weeks | | 3 weeks | | 4 weeks | |
|---|---|---|---|---|---|---|
| | Mean | SD | Mean | SD | Mean | SD |
| ARH77 | 6 | 4 | 13 | 5 | 8 | 4 |
| K562 | 4 | 3 | 6 | 4 | 4 | 1 |

[Table 18]

| NKp30 MFI | 2 weeks | | 3 weeks | | 4 weeks | |
|---|---|---|---|---|---|---|
| | Mean | SD | Mean | SD | Mean | SD |
| ARH77 | 21 | 11 | 16 | 12 | 13 | 10 |
| K562 | 16 | 10 | 8 | 8 | 5 | 3 |

[Table 19]

| NKG2D MFI | 2 weeks | | 3 weeks | | 4 weeks | |
|---|---|---|---|---|---|---|
| | Mean | SD | Mean | SD | Mean | SD |
| ARH77 | 17 | 9 | 17 | 7 | 10 | 4 |
| K562 | 13 | 6 | 10 | 6 | 4 | 1 |

[Table 20]

| DNAM-1 MFI | 2 weeks | | 3 weeks | | 4 weeks | |
|---|---|---|---|---|---|---|
| | Mean | SD | Mean | SD | Mean | SD |
| ARH77 | 14 | 5 | 19 | 10 | 16 | 11 |
| K562 | 11 | 2 | 14 | 2 | 13 | 4 |

As shown in Tables 14 to 20 above and Figs. 16 to 22, ARH77 cells exhibited a higher mean fluorescence intensity (MFI) than K562 cells in all measurements except CD158b, and exhibited a lower mean fluorescence intensity in CD158b, which inhibits the function of NK cells. Therefore, it was confirmed that ARH77 cells express receptors important for NK cell activation better than K562 cells.

Therefore, it can be seen that ARH77 cells have a more excellent NK cell stimulating effect as feeder cells compared to K562 cells.

**[Example 4]**

**NK cell stimulating effect of B7H6, CD137L, IL15, and IL15Rα expressing feeder cells**

In order to confirm the NK cell stimulating effect by B7H6, CD137L, IL-15, and IL15Rα expressing feeder cells, the following experiment was conducted by expressing each gene using ARH77 cells and K562 cells.

**4-1. Evaluation of NK cell expansion rate**

In order to confirm the NK cell stimulating effect by B7H6, CD137L, IL-15, and IL15Rα expressing feeder cells, the expansion rate of NK cells was evaluated using ARH77 cells and K562 cells expressing the above genes.

In the same manner as in Example 1-1 above, PBMCs were isolated and co-cultured with each feeder cell irradiated with radiation. In PBMC prior to culture and each cell recovered on days 14, 21, and 28 of culture, the purity of NK cells (CD3$^-$ CD56$^+$ cells) was evaluated by flow cytometry, and then the expansion rate of NK cells was calculated in the same manner as above.

The experimental results are shown in Table 21 below.

[Table 21]

| Expansion rate (fold) | 2 weeks | | 3 weeks | | 4 weeks | |
|---|---|---|---|---|---|---|
| | Mean | SD | Mean | SD | Mean | SD |
| ARH-77 | 234 | 176 | 1,313 | 1628 | 1,376 | 1,386 |
| ARH-77/B7H6/IL-15 | 99 | 84 | 364 | 419 | 568 | 928 |
| ARH-77/137L/IL-15 | 631 | 356 | 6,910 | 3,931 | 43,448 | 48,524 |
| ARH-77/B7H6/IL-15/IL-15Rα | 116 | 92 | 352 | 238 | 480 | 420 |
| ARH-77/13 7L/IL-15/IL-15Rα | 640 | 388 | 7,391 | 4,684 | 33,231 | 34,541 |
| ARH-77/B7H6/137L/IL-15 | 757 | 386 | 9,821 | 8,477 | 39,732 | 35,137 |
| ARH-77/B7H6/13 7L/IL-15/IL-15Ra | 746 | 360 | 10,030 | 7,358 | 28,983 | 19,224 |
| K562 | 150 | 267 | 477 | 766 | 627 | 798 |
| K562/B7H6/137L/IL-15 | 571 | 342 | 5,398 | 1,368 | 36,353 | 21,864 |
| K562/B7H6/137L/IL-15/IL-15Rα | 748 | 424 | 10,445 | 9,098 | 25,742 | 15,439 |

As shown in Table 21 above, it was confirmed that B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells exhibit the most excellent expansion rate of NK cells at 3 weeks.

**4-2. Evaluation of NK cell purity**

In order to confirm the NK cell stimulating effect by B7H6, CD137L, IL-15, and IL15Rα expressing feeder cells, the purity of NK cells was evaluated using ARH77 cells and K562 cells expressing the above genes.

The purity was evaluated by measuring the ratio of CD3- CD56+ NK cells in the cell by flow cytometry in the same manner as in Example 1-2 above, and the experimental results are shown in Table 22 below.

[Table 22]

| Purity | 2 weeks | | 3 weeks | | 4 weeks | |
|---|---|---|---|---|---|---|
| | Mean | SD | Mean | SD | Mean | SD |
| ARH-77 | 83 | 18 | 78 | 19 | 69 | 20 |
| ARH-77/B7H6/IL-15 | 83 | 15 | 70 | 22 | 60 | 28 |
| ARH-77/137L/IL-15 | 94 | 5 | 92 | 7 | 87 | 12 |
| ARH-77/B7H6/IL-15/IL-15Rα | 83 | 20 | 80 | 22 | 75 | 25 |
| ARH-77/13 7L/IL-15/IL-15Rα | 95 | 5 | 92 | 8 | 86 | 12 |
| ARH-77/B7H6/137L/IL-15 | 95 | 4 | 94 | 5 | 88 | 9 |
| ARH-77/B7H6/13 7L/IL-15/IL-15Ra | 94 | 4 | 93 | 6 | 89 | 9 |
| K562 | 69 | 29 | 60 | 29 | 46 | 27 |
| K562/B7H6/137L/IL-15 | 97 | 3 | 96 | 4 | 93 | 8 |
| K562/B7H6/137L/IL-15/IL-15Rα | 95 | 4 | 95 | 5 | 91 | 7 |

As shown in Table 22 above, it was confirmed that NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, B7H6, CD137L, and IL-15 expressing K562 (K562-B7H6-CD137L-IL15) cell, and B7H6, CD137L, IL-15, and IL-15Rα expressing K562 (K562-B7H6-CD137L-IL15-IL15Rα) cells exhibit a purity of about 90% or more by the fourth week.

### 4-3. Evaluation of NK cell cytotoxicity

In order to confirm the NK cell stimulating effect by B7H6, CD137L, IL15, and IL15Rα expressing feeder cells, the cytotoxicity against cancer cells of NK cells that were proliferated using ARH77 cells and K562 cells expressing the above genes was evaluated.

In the same manner as in Example 1-3 above, the cytotoxicity capability against cancer cells of NK cells that were proliferated using each gene expressing feeder cell was measured, and the experimental results are shown in Table 23 below.

[Table 23]

| Toxicity (%) | 2 weeks | | 3W | | 4W | |
|---|---|---|---|---|---|---|
| | Mean | SD | Mean | SD | Mean | SD |
| ARH-77 | 56 | 13 | 61 | 11 | 50 | 10 |
| ARH-77/B7H6/IL-15 | 60 | 9 | 58 | 11 | 39 | 16 |
| ARH-77/137L/IL-15 | 59 | 10 | 66 | 12 | 50 | 9 |
| ARH-77/B7H6/IL-15/IL-15Rα | 64 | 8 | 67 | 5 | 44 | 15 |
| ARH-77/137L/IL-15/IL-15Rα | 64 | 9 | 63 | 10 | 55 | 11 |
| ARH-77/B7H6/137L/IL-15 | 61 | 13 | 65 | 11 | 65 | 11 |
| ARH-77/B7H6/137L/IL-15/IL-15Rα | 61 | 11 | 64 | 11 | 67 | 14 |
| K562 | 60 | 9 | 62 | 13 | 52 | 17 |
| K562/B7H6/137L/IL-15 | 66 | 8 | 72 | 10 | 55 | 15 |
| K562/B7H6/137L/IL-15/IL-15Rα | 65 | 12 | 65 | 12 | 64 | 13 |

As shown in Table 23 above, it was confirmed that NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells maintain toxicity by the fourth week, but NK cells using the remaining cells have reduced toxicity.

Therefore, it can be seen that B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells have an excellent NK cell stimulating effect as feeder cells.

**[Example 5]**

**NK cell stimulating effect of feeder cells in medium optimized for NK cells**

The following experiment was conducted using B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells, which have an excellent NK cell stimulating effect as feeder cells in Example 3 above.

**5-1. Evaluation of NK cell expansion rate**

In order to confirm the NK cell stimulating effect by the three genetically modified feeder cells, the expansion rate of NK cells was evaluated using a medium optimized for NK cells.

In the same manner as in Example 1-1 above, PBMCs were isolated and co-cultured with each feeder cell irradiated with radiation. As a culture solution, DMEM culture solution containing 30% HAM'S F12, 10% human serum (HS), 10 μg/mL gentamicin, 1x Glutamax, 10 μM β-mercaptoethanol, 50 μM ethanolamine, 20 μg/mL ascorbioc acid, and 5 ng/mL sodium selenite was used. For one week from the start of culture, 10 U/mL recombinant human interleukin (rhIL)-2 and 5 ng/mL rhIL-21 were added to the culture solution, and after 1 week of culture, 100 U/mL rhIL-2 and 5 ng/mL rhIL-15 were added to the culture solution, and cultured for 28 days.

The culture solution was replaced with new culture solution every 1 to 2 days. On days 14, 21, and 28 of culture, the cultured cells were recovered, and the ratio (purity) of NK cells (CD3-, CD56+ cells) in the recovered cells was evaluated by flow cytometry. The total number of NK cells was calculated by multiplying the total number of viable cells among the recovered cells by the ratio of NK cells.

The total number of NK cells in PBMCs prior to culture was calculated in the same manner, and the expansion rate of NK cells was calculated by dividing the total number of NK cells on days 14, 21, and 28 of culture by the number of NK cells prior to culture, respectively.

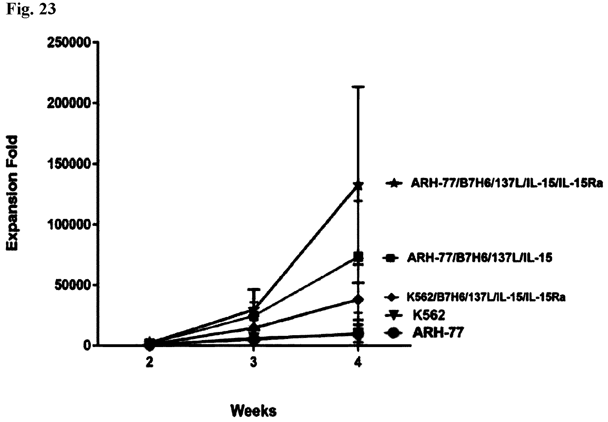

The experimental results are shown in Table 24 below and Fig. 23.

[Table 24]

| Expansion rate (fold) | 2 weeks | | 3 weeks | | 4 weeks | |
|---|---|---|---|---|---|---|
| | Mean | SD | Mean | SD | Mean | SD |
| ARH-77 | 745 | 287 | 5,130 | 2,806 | 9,935 | 7,524 |
| ARH-77/B7H6/137L/IL-15 | 1752 | 876 | 24,539 | 11,282 | 73,276 | 46,272 |
| ARH-77/B7H6/137L/IL-15/IL-15Ra | 2154 | 1,307 | 29,633 | 16,672 | 132,683 | 80,843 |
| K562 | 516 | 531 | 6,057 | 8,017 | 9,513 | 12,002 |
| K562/B7H6/137L/IL-15/IL-15Rα | 872 | 625 | 14,685 | 10,278 | 37,904 | 29,125 |

As shown in Table 24 above and Fig. 23, it was confirmed that B7H6, CD137L, IL-15, and IL-15Rα expressing ARH77 (ARH77-B7H6-CD137L-IL15-IL15Rα) cells have the most excellent effect on NK cell expansion.

Specifically, it was confirmed that NK cells obtained through B7H6, CD137L, IL-15, and IL-15Rα expressing ARH77 (ARH77-B7H6-CD137L-IL15-IL15Rα) cells have an expansion rate by about 3.5 times higher compared to NK cells obtained through B7H6, CD137L, IL-15, and IL-15Rα expressing K562 (K562-B7H6-CD137L-IL15-IL15Rα) cells after 4 weeks of culture.

**5-2. Evaluation of NK cell purity**

In order to confirm the NK cell stimulating effect by the three genetically modified feeder cells of Example 4-1 above, the purity of NK cells was evaluated using a medium optimized for NK cells.

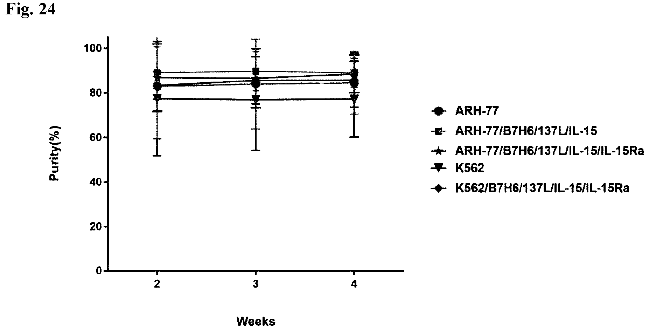

The purity was evaluated by measuring the ratio of CD3- CD56+ NK cells in the cell by flow cytometry in the same manner as in Example 1-2 above, and the experimental results are shown in Table 25 below and Fig. 24.

[Table 25]

| Purity | 2 weeks | | 3 weeks | | 4 weeks | |
|---|---|---|---|---|---|---|
| | Mean | SD | Mean | SD | Mean | SD |
| ARH-77 | 83 | 24 | 84 | 20 | 85 | 14 |
| ARH-77/B7H6/137L/IL-15 | 89 | 12 | 90 | 9 | 89 | 7 |
| ARH-77/B7H6/137L/IL-15/IL-15Ra | 87 | 15 | 87 | 13 | 89 | 9 |
| K562 | 77 | 26 | 77 | 23 | 77 | 17 |
| K562/B7H6/137L/IL-15/IL-15Rα | 83 | 6 | 86 | 11 | 86 | 12 |

As shown in Table 25 above and Fig. 24, it was confirmed that NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells all maintain a purity of about 85% or more for up to 4 weeks. In particular, it was confirmed that NK cells obtained through ARH77 cells have a more excellent purity.

**5-3. Evaluation of NK cell cytotoxicity**

In order to confirm the NK cell stimulating effect by the three genetically modified feeder cells, the cytotoxicity ofNK cells was evaluated using a medium optimized for NK cells.

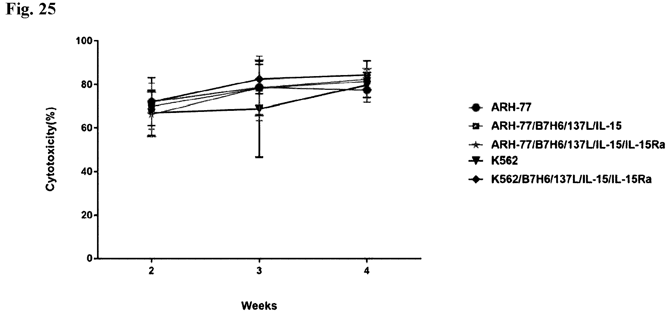

The cytotoxicity against cancer cells of NK cells that were proliferated using each gene expressing feeder cell was measured in the same manner as in Example 1-3 above, and the experimental results are shown in Table 26 below and Fig. 25.

[Table 26]

| Toxicity (%) | 2 weeks | | 3 weeks | | 4 weeks | |
|---|---|---|---|---|---|---|
| | Mean | SD | Mean | SD | Mean | SD |
| ARH-77 | 72 | 5 | 79 | 12 | 78 | 6 |
| ARH-77/B7H6/137L/IL-15 | 70 | 11 | 78 | 15 | 82 | 5 |
| ARH-77/B7H6/137L/IL-15/IL-15Ra | 66 | 10 | 79 | 13 | 83 | 5 |
| K562 | 67 | 10 | 69 | 22 | 80 | 6 |
| K562/B7H6/137L/IL-15/IL-15Rα | 72 | 11 | 83 | 7 | 84 | 7 |

As shown in Table 26 above and Fig. 25, it was confirmed that NK cells obtained through B7H6, CD137L, and IL-15 expressing ARH-77 (ARH77-B7H6-CD137L-IL15) cells, B7H6, CD137L, IL-15, and IL-15Rα expressing ARH77 (ARH77-B7H6-CD137L-IL15-IL15Rα) cells, and B7H6, CD137L, IL-15, and IL-15Rα expressing K562 (K562-B7H6-CD137L-IL15-IL15Rα) cells all exhibit excellent cytotoxicity.

Ultimately, from the above experimental results, it can be seen that B7H6, CD137L, IL-15, and IL-15Rα expressing ARH77 (ARH77-B7H6-CD137L-IL15-IL15Rα) cells have the most excellent NK cell stimulating effect as feeder cells.

**Claims**

1. A composition for proliferating natural killer cells, comprising feeder cells that express B7H6.

2. The composition for proliferating natural killer cells according to claim 1, wherein the composition further expresses at least one selected from the group consisting of CD137L, IL-15, and IL-15Rα.

3. The composition for proliferating natural killer cells according to claim 1, wherein the composition comprises feeder cells that express B7H6, CD137L, and IL-15.

4. The composition for proliferating natural killer cells according to claim 1, wherein the composition comprises feeder cells that express B7H6, CD137L, IL-15, and IL-15Rα.

5. The composition for proliferating natural killer cells according to claim 1, wherein the feeder cells are ARH77 or K562 cells.

6. The composition for proliferating natural killer cells according to claim 1, wherein the feeder cells are ARH77 cells.

7. A feeder cell line that expresses B7H6.

8. The feeder cell line according to claim 7, wherein the feeder cell line further expresses at least one selected from the group consisting of CD137L, IL-15, and IL-15Rα.

9. The feeder cell line according to claim 7, wherein the feeder cell line is ARH77 or K562 cell line.

10. A method of producing feeder cells that express B7H6, comprising the steps of:

(1) transforming the B7H6 gene with a virus expression vector to produce a recombinant virus; and
(2) adding the recombinant virus to the feeder cells and culturing them.

11. The method of producing feeder cells according to claim 10, wherein in step (1), at least one gene selected from the group consisting of CD137L, IL-15, and IL-15Rα is further transformed.

12. The method of producing feeder cells according to claim 10, wherein the feeder cells are ARH77 or K562 cells.

13. The method of producing feeder cells according to claim 10, wherein the virus is a lentivirus or a retrovirus.

14. A method for proliferating natural killer cells, comprising the step of culturing feeder cells that express B7H6 and peripheral blood mononuclear cells.

15. The method for proliferating natural killer cells according to claim 14, wherein the feeder cells further express at least one selected from the group consisting of CD137L, IL-15, and IL-15Rα.

16. The method for proliferating natural killer cells according to claim 14, wherein the feeder cells are ARH77 or K562 cells.

17. The method for proliferating natural killer cells according to claim 14, wherein the culture solution comprises at least one selected from the group consisting of penicillin, streptomycin, glutamine, gentamicin, fetal bovine serum, human serum, mercaptoethanol, ethanolamine, ascorbic acid, and sodium selenite.

18. The method for proliferating natural killer cells according to claim 14, wherein the culture solution comprises at least one selected from the group consisting of glutamine, gentamicin, human serum, mercaptoethanol, ethanolamine, ascorbic acid, and sodium selenite.

19. The method for proliferating natural killer cells according to claim 14, wherein the method further comprises the step of adding at least one selected from the group consisting of recombinant human interleukin-2, recombinant human interleukin-21, and recombinant human interleukin-15.

20. The method for proliferating natural killer cells according to claim 14, wherein the method further comprises the steps of (a) adding recombinant human interleukin-2 and recombinant human interleukin-21, and (b) adding recombinant human interleukin-2 and recombinant human interleukin-15.

21. A natural killer cell line produced by the method according to claim 14.

Fig. 1

Fig. 2a

**Fig. 2b**

⑤ LV-cIL-15 & LV-cIL15-Ra
Co-Transduction

⑦ Sorted ARH-77 (CD137L/B7H6/IL-15/IL15Ra)

Fig. 3

Fig. 4

Fig. 5

Fig. 6

Fig. 7

Fig. 8

**Fig. 9**

## Expansion Fold

**Fig. 10**

## Purity

**Fig. 11**

Cytotoxicity

Fig. 12

Fig. 13

Fig. 14

PBMC (Day 0)    ARH-77    K562

Fig. 15

Fig. 16

**NKp46**

Fig. 17

**CD16**

Fig. 18

## CD158b

- ■ 2 WEEKS
- ■ 3 WEEKS
- ▨ 4 WEEKS

Fig. 19

## NKp44

- ■ 2 WEEKS
- ■ 3 WEEKS
- ▨ 4 WEEKS

Fig. 20

## NKp30

- ■ 2 WEEKS
- ■ 3 WEEKS
- ▨ 4 WEEKS

Fig. 21

## NKG2D

- 2 WEEKS
- 3 WEEKS
- 4 WEEKS

Fig. 22

## DNAM-1

- 2 WEEKS
- 3 WEEKS
- 4 WEEKS

Fig. 23

Fig. 24

Legend:
- ARH-77
- ARH-77/B7H6/137L/IL-15
- ARH-77/B7H6/137L/IL-15/IL-15Ra
- K562
- K562/B7H6/137L/IL-15/IL-15Ra

Fig. 25

## INTERNATIONAL SEARCH REPORT

| International application No. |
|---|
| **PCT/KR2022/007782** |

**A.      CLASSIFICATION OF SUBJECT MATTER**

**C12N 5/0783**(2010.01)i; **C07K 14/47**(2006.01)i; **C07K 14/705**(2006.01)i; **C07K 14/54**(2006.01)i; **C07K 14/715**(2006.01)i; **C12N 5/09**(2010.01)i

According to International Patent Classification (IPC) or to both national classification and IPC

**B.      FIELDS SEARCHED**

Minimum documentation searched (classification system followed by classification symbols)

C12N 5/0783(2010.01); A61K 35/18(2006.01); C12N 13/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 배양보조세포(feeder cells), B7H6, 자연살해세포(natural killer cell), CD137L, IL-15, IL-15Rα, 말초혈액단핵구(peripheral blood mononuclear cell, PBMC)

**C.      DOCUMENTS CONSIDERED TO BE RELEVANT**

| Category* | Citation of document, with indication, where appropriate, of the relevant passages | Relevant to claim No. |
|---|---|---|
| X | CN 103484429 A (QINGDAO MAIDISAISI BIOLOGICAL SCIENCE & TECHNOLOGY CO., LTD.) 01 January 2014 (2014-01-01)<br>    See abstract; claims 1-7; and paragraphs [0006] and [0022]-[0025]. | 1-3,5-21 |
| Y |  | 4 |
| Y | KR 10-2019-0135912 A (SAMSUNG LIFE PUBLIC WELFARE FOUNDATION et al.) 09 December 2019 (2019-12-09)<br>    See claims 1-8; and paragraph [0048]. | 4 |
| Y | KR 10-2020-0140279 A (RUBIUS THERAPEUTICS, INC.) 15 December 2020 (2020-12-15)<br>    See abstract; claims 1-5, 28-29 and 37-38; and paragraph [0024]. | 4 |
| A | KR 10-2020-0001595 A (GREEN CROSS LAB CELL CORPORATION) 06 January 2020 (2020-01-06)<br>    See entire document. | 1-21 |

[✓] Further documents are listed in the continuation of Box C.      [✓] See patent family annex.

| | |
|---|---|
| *      Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **05 September 2022** | **05 September 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
|---|
| **PCT/KR2022/007782** |

**C.    DOCUMENTS CONSIDERED TO BE RELEVANT**

| Category* | Citation of document, with indication, where appropriate, of the relevant passages | Relevant to claim No. |
|---|---|---|
| A | BRANDT, C. S. et al. The B7 family member B7-H6 is a tumor cell ligand for the activating natural killer cell receptor NKp30 in humans. J. Exp. Med. 2009, vol. 206, no. 7, pp. 1495-1503.<br>See entire document. | 1-21 |

Form PCT/ISA/210 (second sheet) (July 2019)

**INTERNATIONAL SEARCH REPORT**

International application No.

**PCT/KR2022/007782**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

42

**INTERNATIONAL SEARCH REPORT**
Information on patent family members

International application No.

**PCT/KR2022/007782**

| Patent document cited in search report | | | Publication date (day/month/year) | Patent family member(s) | | | Publication date (day/month/year) |
|---|---|---|---|---|---|---|---|
| CN | 103484429 | A | 01 January 2014 | None | | | |
| KR | 10-2019-0135912 | A | 09 December 2019 | KR | 10-2227155 | B1 | 12 March 2021 |
| | | | | WO | 2019-231243 | A1 | 05 December 2019 |
| KR | 10-2020-0140279 | A | 15 December 2020 | AU | 2019-232012 | A1 | 24 September 2020 |
| | | | | CA | 3093387 | A1 | 12 September 2019 |
| | | | | CN | 112105723 | A | 18 December 2020 |
| | | | | EP | 3762422 | A1 | 13 January 2021 |
| | | | | IL | 277159 | A | 29 October 2020 |
| | | | | JP | 2021-514665 | A | 17 June 2021 |
| | | | | SG | 11202008261 | A | 29 September 2020 |
| | | | | US | 11141433 | B2 | 12 October 2021 |
| | | | | US | 2019-0298769 | A1 | 03 October 2019 |
| | | | | WO | 2019-173798 | A1 | 12 September 2019 |
| KR | 10-2020-0001595 | A | 06 January 2020 | AU | 2018-271755 | A1 | 21 November 2019 |
| | | | | AU | 2018-271755 | B2 | 23 December 2021 |
| | | | | CA | 3062130 | A1 | 22 November 2019 |
| | | | | CN | 110662834 | A | 07 January 2020 |
| | | | | EP | 3633029 | A2 | 08 April 2020 |
| | | | | JP | 2020-521462 | A | 27 July 2020 |
| | | | | JP | 7039623 | B2 | 22 March 2022 |
| | | | | US | 2020-0108096 | A1 | 09 April 2020 |
| | | | | WO | 2018-217064 | A2 | 29 November 2018 |
| | | | | WO | 2018-217064 | A3 | 28 March 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HIROYUKI FUJISAKI ; HARUMI KAKUDA ; NORIKO SHIMASAKI ; CHIHAYA IMAI ; JING MA ; TIMOTHY LOCKEY et al.** EXPANSION OF HIGHLY CYTOTOXIC HUMAN NATURAL KILLER CELLS FOR CANCER CELL THERAPY. *Cancer Res. Author manuscript.,* 01 May 2009, vol. 69 (9), 4010-4017

- **DENMAN CJ ; SENYUKOV VV ; SOMANCHI SS ; PHATARPEKAR PV ; KOPP LM et al.** Membrane-Bound IL-21 Promotes Sustained Ex Vivo Proliferation of Human Natural Killer Cells. *PLoS ONE,* 2012, vol. 7 (1), e30264